Europäisches Patentamt

European Patent Office

Office européen des brevets

(19)

(11) Numéro de publication: **0 228 968**
**B1**

(12)

# FASCICULE DE BREVET EUROPEEN

(45) Date de publication du fascicule du brevet:
**25.04.90**

(51) Int. Cl.⁴: **A61M 1/16**

(21) Numéro de dépôt: **86402924.4**

(22) Date de dépôt: **23.12.86**

(54) **Procédé et dispositif pour le dégazage et le chauffage simultanés de l'eau utilisée pour préparer un liquide de dialyse.**

(30) Priorité: **24.12.85 FR 8519151**

(43) Date de publication de la demande:
**15.07.87 Bulletin 87/29**

(45) Mention de la délivrance du brevet:
**25.04.90 Bulletin 90/17**

(84) Etats contractants désignés:
**DE GB IT SE**

(56) Documents cités:
**FR-A- 2 073 337**

(73) Titulaire: **MATERIEL MEDICAL FRANCE Société Anonyme dite:, Domaine du Bois du Maine-Sud, F-69210 Savigny(FR)**

(72) Inventeur: **Mion, Jean, 49, rue de Sèze, F-69006 Lyon(FR)**

(74) Mandataire: **Jolly, Jean-Pierre et al, Cabinet BROT et JOLLY 83, rue d'Amsterdam, F-75008 Paris(FR)**

ACTORUM AG

## Description

La présente invention concerne la préparation des liquides utilisés dans les traitements d'hémodialyse. Elle concerne plus particuliérement un procédé et un dispositif destinés à dégazer et à chauffer simultanément l'eau que l'on emploie pour préparer le dialysat dans un système de dialyse à circuit ouvert.

Habituellement, les liquides de dialyse sont préparés en dissolvant dans de l'eau préalablement chauffée des solutions concentrées de divers sels dites "électrolytes". Le liquide de dialyse doit être sensiblement à la température du sang du patient traité et il faut donc porter l'eau que l'on emploie de la température ambiante à celle du patient, c'est-à-dire élever sa température de quelques degrés centigrades à environ 40°C. Ce chauffage se traduit toutefois par le passage en phase gazeuse des gaz dissous dans l'eau, en particulier de l'air, et il est nécessaire d'éliminer ces gaz avant l'introduction du liquide dans le dialyseur, car l'oxygéne agit comme un poison vis-à-vis de la membrane semi-perméable du dialyseur, qu'il neutralise.

Le dégazage de l'eau fait généralement appel à deux types de technique:
- soit on soumet l'eau à une pression inférieure à la pression atmosphérique, de l'ordre de 850 millibars par exemple, à la température d'utilisation (environ 40°C);
- soit on chauffe l'eau à une haute température, de l'ordre de 95°C, à la pression atmosphérique.

Le premier processus consomme peu d'énergie, mais il est d'une faible efficacité. Au contraire, la seconde technique se révèle très efficace, mais elle est grande consommatrice d'énergie, même si l'on récupère par l'intermédiaire d'un échangeur de chaleur les calories que contient le dialysat usé, avant de rejeter celui-ci.

La présente invention fait appel à la seconde technique, c'est-à-dire le chauffage de l'eau à température élevée et vise à limiter la quantité d'énergie calorifique ainsi consommée.

Pour limiter les pertes d'énergie, FR - A - 2 073 337 a proposé un système de chauffage et de dégazage de l'eau servant à la préparation d'un liquide de dialyse, dans lequel l'eau est préchauffée dans un premier échangeur par échange thermique avec le dialysat usé rejeté, puis est ensuite chauffée, à nouveau par échange thermique, dans un second échangeur, avec la ligne de sortie d'un élément de dégazage, traverse alors un élément de chauffage et est enfin traitée dans l'élément de dégazage. C'est toutefois la totalité du dialysat usé chaud qui est utilisée pour préchauffer l'eau froide, dans le premier échangeur, sans aucune possibilité de régler la quantité de chaleur ainsi transférée.

Or, si le dégazage thermique d'un liquide est très efficace, il est actuellement très peu pratiqué dans le domaine de la dialyse, car il nécessite des échangeurs thermiques dont on puisse contrôler parfaitement le fonctionnement. En effet, le dégazage s'effectue à une température fixe et il est donc nécessaire de contrôler avec précision l'échange thermique dans le second échangeur immédiatement en amont du moyen de chauffage et du dispositif de dégazage. Il faut par conséquent régler la température d'entrée de l'eau à chauffer dans cet échangeur en fonction de la température de la ligne d'eau chaude sortant dudit échangeur. Dans ce but, conformément à l'invention, on propose d'ajuster simplement, à l'aide d'une vanne, la quantité de dialysat usé constituant la phase chaude dans le premier échangeur, sans qu'il soit nécessaire d'utiliser des échangeurs compliqués.

L'invention a par conséquent pour objet un procédé de dégazage et de chauffage simultanés de l'eau utilisée pour préparer en continu le dialysat d'un système de dialyse à circuit ouvert, dans lequel l'eau est d'abord préchauffée dans un premier échangeur thermique par échange thermique avec au moins une partie du dialysat usé chaud rejeté par ledit système de dialyse, est ensuite chauffée à nouveau par échange thermique dans un second échangeur thermique avec l'eau chaude provenant du système de dégazage et est enfin chauffée par un moyen de chauffage avant d'être traitée dans ledit système de dégazage, procédé caractérisé en ce que l'on règle la quantité de dialysat usé chaud soumise dans le premier échangeur à un échange thermique avec l'eau à préchauffer de façon telle que la température de l'eau chaude dégazée alimentant le système de dialyse demeure égale à une valeur de consigne.

Avec la vanne de régulation disposée en amont du premier échangeur thermique, sur le circuit d'alimentation en dialysat usé de cet échangeur, il est ainsi possible, en ajustant la fraction de dialysat usé qui le traverse, de régler la température de l'eau à l'entrée de l'échangeur suivant. Avantageusement, cette vanne de régulation sera équipée d'un système de commande asservi à une sonde de température disposée sur le circuit d'eau en aval du circuit chaud du second échangeur thermique, c'est-à-dire sur la ligne d'alimentation du dialyseur, en vue de maintenir à une valeur préfixée la température de l'eau à l'emplacement de ladite sonde.

L'invention a donc également pour objet, dans un système de dialyse, un dispositif pour le dégazage et le chauffage simultanés de l'eau utilisée pour préparer un liquide de dialyse, du type comprenant un moyen de chauffage apte à amener l'eau à une température suffisante pour pouvoir effectuer un dégazage efficace sensiblement à la pression atmosphérique et un moyen de dégazage de l'eau ainsi chauffée, ce dispositif comprenant sur le circuit d'eau, en amont dudit moyen de chauffage, un premier échangeur thermique dont le circuit froid est emprunté par l'eau à chauffer et dont le circuit chaud est alimenté par une partie au moins du dialysat usé en provenance du dialyseur, l'eau provenant de ce premier échangeur traversant ensuite un second échangeur thermique dont le circuit chaud est alimenté par l'eau chaude en provenance dudit moyen de dégazage avant qu'elle n'accède au circuit d'alimentation du dialyseur, ce dispositif étant caractérisé en ce qu'il comporte, sur le circuit d'alimentation en dialysat usé chaud dudit premier échangeur thermique un moyen de réglage de la quantité du dialysat usé admis dans le circuit chaud de cet échangeur, le moyen de réglage étant asservi à la température de l'eau chaude dégazée alimentant le système de dialyse et apte à maintenir cette température égale à une valeur de consigne.

Avantageusement, une tuyère, un tube de Venturi ou similaire, sera interposé sur le circuit d'eau, entre le second échangeur thermique et le moyen de dégazage, afin d'abaisser la pression statique de l'eau et de faciliter son dégazage.

L'élément chauffant sera de préférence asservi à une sonde de température disposée sur le circuit d'eau entre cet élément et le dispositif de dégazage pour maintenir la température de l'eau sortant de cet élément de chauffage à une valeur préfixée. Il est ainsi possible de chauffer l'eau systématiquement à 100°C par ce moyen de chauffage, pour obtenir un dégazage complet de cette eau.

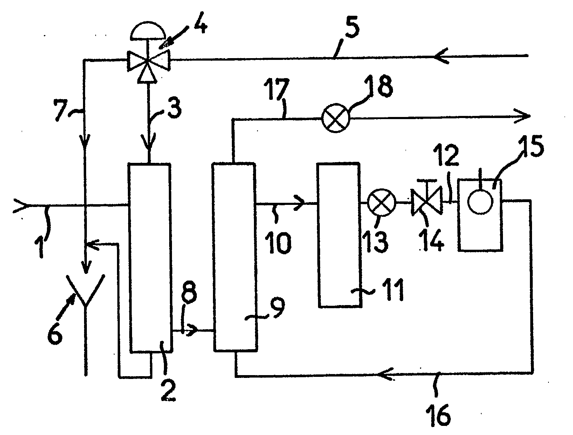

Le dessin schématique annexé, qui n'a pas de caractère limitatif, illustre une forme de mise en oeuvre de l'invention. Sur ce dessin, seuls les organes du système de dialyse intéressant la présente invention ont été représentés.

L'eau à chauffer et à dégazer est amenée par une ligne 1 à un échangeur thermique 2, dont le circuit chaud est alimenté en dialysat usé chaud, en provenance du dialyseur, par une ligne 3, connectée par une vanne à trois voies 4 à la ligne 5 d'évacuation du dialysat usé, dont une partie peut être rejetée directement à l'égout 6 par la ligne 7.

L'eau ainsi préchauffée est ensuite acheminée par la ligne 8 à un échangeur thermique 9, dont le mode d'alimentation du circuit chaud sera indiqué ci-après. L'eau à nouveau chauffée dans l'échangeur est évacuée par la ligne 10 en direction d'un élément chauffant 11, qui amène sa température à environ 100°C, puis par une ligne 12, équipée d'une sonde de température 13, à laquelle est asservi l'élément chauffant 11, et d'un venturi 14, destiné à abaisser la pression statique de l'eau, à un organe de dégazage 15.

Après dégazage complet dans l'organe 15, l'eau est évacuée par la ligne 16 à l'alimentation du circuit chaud de l'échangeur 9, puis par la ligne 17, équipée d'une sonde de température 18 à laquelle est asservie la vanne 4, vers le système de préparation du dialysat frais et d'alimentation du dialyseur.

Les sondes 13 et 18 sont connectées à un poste de commande central à pilotage automatique, qui commande la vanne 4 et le moyen de chauffage 11, comme indiqué ci-dessus, de manière à maintenir à une valeur de consigne les températures mesurées par les sondes 13 et 18. Le rôle de la vanne 4 est important, car moyennant un réglage adéquat du moyen de chauffage 11, la vanne 4 peut permettre à elle seule de contrôler le bilan thermique de l'ensemble du dispositif.

L'invention apporte donc un moyen simple pour dégazer complètement l'eau destinée à préparer le dialysat frais d'un système de dialyse à circuit ouvert et pour chauffer simultanément cette eau sans consommation excessive d'énergie thermique.

## Revendications

1. Procédé de dégazage et de chauffage simultanés de l'eau utilisée pour préparer en continu le dialysat d'un système de dialyse à circuit ouvert, dans lequel l'eau est d'abord préchauffée dans un premier échangeur thermique par échange thermique avec au moins une partie du dialysat usé chaud rejeté par ledit système de dialyse, est ensuite chauffée à nouveau par échange thermique dans un second échangeur thermique avec l'eau chaude provenant du système de dégazage et est enfin chauffée par un moyen de chauffage avant d'être traitée dans ledit système de dégazage, procédé caractérisé en ce que l'on règle la quantité de dialysat usé chaud soumise dans le premier échangeur à un échange thermique avec l'eau à préchauffer de façon telle que la température de l'eau chaude dégazée alimentant le système de dialyse demeure égale à une valeur de consigne.

2. Dans un système de dialyse, un dispositif pour le dégazage et le chauffage simultanés de l'eau ùtilisée pour préparer un liquide de dialyse, du type comprenant un moyen de chauffage (11) apte à amener l'eau à une température suffisante pour pouvoir effectuer un dégazage efficace sensiblement à la pression atmosphérique et un moyen de dégazage (15) de l'eau ainsi chauffée, ce dispositif comprenant sur le circuit d'eau, en amont dudit moyen de chauffage, un premier échangeur thermique (2) dont le circuit froid (1) est emprunté par l'eau à chauffer et dont le circuit chaud (3) est alimenté par une partie au moins du dialysat usé en provenance du dialyseur, l'eau provenant de ce premier échangeur traversant ensuite un second échangeur thermique (9) dont le circuit chaud (16) est alimenté par l'eau chaude en provenance dudit moyen de dégazage (15), avant qu'elle n'accède au circuit d'alimentation (17) du dialyseur, ce dispositif étant caractérisé en ce qu'il comporte, sur le circuit d'alimentation en dialysat usé chaud dudit premier échangeur thermique (2) un moyen de réglage (4) de la quantité du dialysat usé admis dans le circuit chaud de cet échangeur, le moyen de réglage (4) étant asservi à la température de l'eau chaude dégazée alimentant le système de dialyse et apte à maintenir cette température égale à une valeur de consigne.

3. Dispositif selon la revendication 2, caractérisé en ce que ledit moyen de réglage comprend une vanne (4) asservie à une sonde (18) de mesure de la température de l'eau chaude dégazée alimentant le dialyseur.

4. Dispositif selon l'une des revendications 2 et 3, caractérisé en ce que ledit moyen de chauffage (11) est équipé d'un système de commande asservi à une sonde de température (13) disposée sur le circuit d'eau entre ledit moyen de chauffage (11) et le moyen de dégazage (15).

5. Dispositif selon l'une des revendications 2 à 4, caràctérisé en ce qu'un moyen (14) propre à abaisser la pression statique de l'eau est interposé entre ledit élément chauffant (11) et ledit moyen de dégazage (15).

**Patentansprüche**

1. Verfahren zum gleichzeitigen Entgasen und Erwärmen von Wasser, das für die fortlaufende Zubereitung des Dialysats eines Dialysesystems mit offenem Kreislauf verwendet wird, bei welchem das Wasser zunächst in einem ersten Wärmetauscher durch Wärmetausch mit mindestens einem Teil des Dialysats vorerwärmt wird, das warm nach Benutzung durch das Dialysesystem zuzückgegeben wird, und in welchem das Wasser dann erneut durch Wärmetausch in einem zweiten Wärmetauscher mit warmem Wasser erwärmt wird, das von einem Entgasungssystem stammt, und in welchem das Wasser schließlich durch eine Heizeinrichtung erwärmt wird, bevor es in dem Entgasungssystem behandelt wird, dadurch gekennzeichnet, daß man die Menge des warmen benutzten Dialysats, die in dem ersten Wärmetauscher einem Wärmetausch mit dem vorzuerwärmenden Wasser unterworfen ist, derart regelt, daß die Temperatur des entgasten warmen Wassers, das das Dialysesystem versorgt, bei einem Sollwert gleich bleibt.

2. In einem Dialysesystem, eine Vorrichtung für die gleichzeitige Entgasung und Erwärmung des für das Bereitstellen einer Dialyseflüssigkeit verwendeten Wassers, derart, daß sie eine Heizeinrichtung (11), die geeignet ist, Wasser mit einer Temperatur zu erzeugen, die ausreicht, um eine wirksame Entgasung, insbesondere bei atmosphärischem Druck, bewirken zu können, und eine Entgasungseinrichtung (15) des so erwärmten Wassers aufweist, welche Entgasungseinrichtung in dem Wasserkreis stromauf der Heizeinrichtung einen ersten Wärmetauscher (2) aufweist, von dem der Kaltkreislauf (1) durch das zu erwärmende Wasser benutzt wird und von dem der Warmkreislauf (3) durch mindestens einen Teil des aus dem Dialysegerät stammenden benutzten Dialysats versorgt wird, wobei das aus dem ersten Wärmetauscher stammende Wasser anschließend einen zweiten Wärmetauscher (9) durchtritt, von dem der Warmkreislauf (16) durch das warme Wasser, das von der Entgasungseinrichtung (15) stammt, versorgt wird, bevor dieses in den Versorgungskreislauf (17) des Dialysegeräts eingetreten ist, dadurch gekennzeichnet, daß die Vorrichtung im Speisekreislauf des warmen benutzten Dialysats des ersten Wärmetauschers (2) eine Regelvorrichtung (4) für die Menge des verwendeten Dialysats aufweist, das in den Warmkreislauf des Wärmetauschers hineingelassen wird, wobei die Regelvorrichtung (4) von der Temperatur des warmen entgasten Wassers gesteuert wird, das das Dialysesystem versorgt und so ausgelegt ist, daß sie diese Temperatur auf einem Sollwert gleichhalten kann.

3. Vorrichtung nach Anspruch 2, dadurch gekennzeichnet, daß die Regelvorrichtung einen Schieber oder eine Klappe (4) aufweist, die von einer Meßsonde (18) für die Temperatur des warmen entgasten, das Dialysegerät versorgenden Wassers gesteuert wird.

4. Vorrichtung nach einem der Ansprüche 2 und 3, dadurch gekennzeichnet, daß die Heizvorrichtung (11) mit einem Befehlssystem ausgerüstet ist, das von einer Temperatursonde (13) gesteuert ist, die in dem Wasserkreislauf zwischen der Heizeinrichtung (11) und der Entgasungseinrichtung (15) angeordnet ist.

5. Vorrichtung nach einem der Ansprüche 2 und 4, dadurch gekennzeichnet, daß ein Mittel (14), das geeignet ist, den statischen Druck des Wassers zu vermindern, zwischen dem Heizelement (11) und der Entgasungseinrichtung (15) angeordnet ist.

**Claims**

1. A process for the simultaneous degassing and heating of the water used to prepare continuously the dialysate of an open circuit dialysis system, in which the water is firstly preheated in a first heat exchanger by heat exchange with at least part of the hot spent dialysate discharged by said dialysis system, is then heated again by heat exchange in a second heat exchanger with the hot water coming from the degassing system and is finally heated by a heating means before being treated in said degassing system, said process being characterized in that the quantity of spent hot dialysate subjected in the first exchanger to heat exchange with the water to be preheated is regulated in such a way that the temperature of the degassed hot water supplying the dialysis system remains equal to a desired value.

2. In a dialysis system, a device for simultaneously degassing and heating the water used to prepare a dialysis liquid, of the type comprising a heating means (11) capable of bringing the water to a temperature sufficient to be able to carry out effective degassing substantially at atmospheric pressure and a means (15) of degassing the water thus heated, this device comprising in the water circuit, upstream of said heating means, a first heat exchanger (2) of which the cold circuit (1) is made use of by the water to be heated and of which the hot circuit (5) is supplied with part at least of the spent dialysate coming from the dialyser, the water coming from this first exchanger then passing through a second heat exchanger (9) of which the hot circuit (16) is supplied with the hot water coming from said degassing means (15) before it reaches the dialyser supply circuit (17), said device being characterized in that it comprises, in the spent hot dialysate supply circuit of said first heat exchanger (2), a means (4) of regulating the amount of spent dialysate admitted into the hot circuit of said exchanger, the regulating means (4) being controlled by the temperature of the degassed hot water supplying the dialysis system and capable of keeping this temperature equal to a desired value.

3. A device according to claim 2, characterized in that said regulating means comprises a valve (4) controlled by a sensor (18) for measuring the temperature of the degassed hot water supplying the dialyser.

4. A device according to either one of claims 2 or 3, characterized in that said heating means (11) is equipped with a control system controlled by a temperature sensor (13) arranged in the water circuit between said heating means (11) and the degassing means (15).

5. A device according to any one of claims 2 to 4, characterized in that a means (14) suitable for lowering the static pressure of the water is interposed between said heating element (11) and said degassing means (15).